# EUROPEAN PATENT APPLICATION

(11) **EP 4 674 966 A1**
(43) Date of publication of application: **07.01.2026**
(21) Application number: 24763926.3
(22) Date of filing: 27.02.2024
(51) Int. Cl.: C12N 15/52, A61K 31/405, A61K 38/16, A61K 48/00, A61P 3/10, A61P 9/10, A61P 25/00, A61P 25/16, A61P 25/28, A61P 35/00, A61P 43/00, C12M 1/00, C12N 15/10, C12N 15/11, C12N 15/13, C12N 15/29, C12N 15/62, C12N 15/80, C12N 15/81, C12N 15/85

(54) **METHOD FOR DEGRADING TARGET PROTEIN**

(30) Priority: 01.03.2023 JP 2023030923
(71) Applicant: Inter-University Research Institute Corporation Research Organization of Information and Systems, Tokyo 190-8562 (JP)
(72) Inventor: KANEMAKI, Masato, Mishima-shi, Shizuoka 411-8540 (JP)
(74) Representative: HGF
(86) International application number: PCT/JP2024/007114
(87) International publication number: WO 2024/181447

(57) **Abstract**

A fusion protein for use in an auxin-degron system for controlling degradation of a target protein in a non-plant-derived eukaryotic cell, the fusion protein containing a degradation tag that contains at least a part of an Aux/IAA family protein and has an affinity for a complex of a TIR1 family protein with an auxin or an auxin analog, and a protein having an affinity for the target protein.

## Description

### TECHNICAL FIELD

The present invention relates to a method for degrading a target protein. Specifically, the present invention relates to a method for degrading a target protein using an auxin-degron system that controls degradation of a target protein in a non-plant-derived eukaryotic cell.

### BACKGROUND ART

The present inventors have been developing a protein degradation control technology called an auxin-degron system (see, for example, Patent Documents 1 to 3). In this system, a TIR1 that constitutes an auxin-responsive ubiquitin ligase is introduced into cells derived from a eukaryote such as a yeast and animal cells, and the degradation of a target protein to which a degradation tag (a plant-derived Aux/IAA family protein or a partial protein thereof; also referred to as a degron) has been added is controlled by adjusting the presence or absence of an auxin, or an addition timing of the auxin.

The degradation control method for a target protein (also referred to as an auxin-degron method) developed by the present inventors has already been widely used in cell biology research, and is also being applied to model organisms such as yeast, nematode, fruit fly, zebrafish, and mouse.

### Citation List

### Patent Documents

Patent Document 1: Japanese Unexamined Patent Application, First Publication No. 2008-187958
Patent Document 2: PCT International Publication No. WO2010/125620
Patent Document 3: PCT International Publication No. WO2013/073653

### SUMMARY OF INVENTION

### Technical Problem

In the auxin-degron method in the related art, it is necessary to add a degradation tag to a target protein in a cell by genome editing or the like. In patients or diseased animals, it is difficult to add a degradation tag. Therefore, there is room for improvement in clinical applications of the auxin-degron system.

The present invention has been made in view of the above circumstances, and provides a method for degrading a target protein, which can control degradation of a protein without adding a degradation tag to an endogenous target protein.

### Solution to Problem

The present invention includes the following aspects.
[1] A kit of an auxin-degron system for controlling degradation of a protein of interest in a non-plant-derived eukaryotic cell, the kit including: a first nucleic acid encoding a TIR1 family protein; an auxin or an auxin analog having an affinity for the TIR1 family protein; a second nucleic acid encoding a fusion protein containing a degradation tag that contains at least a part of an Aux/IAA family protein and has an affinity for a complex of a TIR1 family protein with an auxin or an auxin analog, and a protein having an affinity for the target protein.
[2] The kit according to [1], in which the protein having the affinity for the target protein is a protein domain that binds to the target protein.
[3] The kit according to [2], in which the protein domain that binds to the target protein is a single-chain antibody.
[4] The kit according to any one of [1] to [3], in which the fusion protein is ubiquitination-resistant.
[5] The kit according to any one of [1] to [4], in which the TIR1 family protein is a rice-derived protein.
[6] The kit according to any one of [1] to [5], in which the TIR1 family protein is a mutant TIR1 family protein having a mutation at an auxin binding site.
[7] The kit according to [6], in which the mutant TIR1 family protein is a protein in which F at a position 74 of OsTIR1 has been mutated into A, G, or S.
[8] The kit according to any one of [1] to [7], in which the auxin analog is a compound represented by General Formula (I) or an ester thereof. 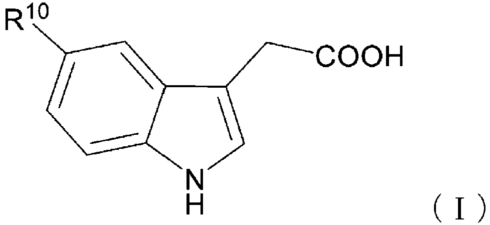 (In General Formula (I), R¹⁰ is a cyclic aliphatic hydrocarbon group which may have a substituent and in which a part of carbon atoms constituting a ring may be substituted with a heteroatom, or an aromatic hydrocarbon group which may have a substituent and in which a part of carbon atoms constituting a ring may be substituted with a heteroatom.)
[9] The kit according to any one of [1] to [8], further including a third nucleic acid encoding a linker linked between the first nucleic acid and the second nucleic acid for controlling, by one promoter, a plurality of genes.
[10] The kit according to any one of [1] to [9] for use in a treatment of a disease, in which the kit is for simultaneous or sequential use in the treatment.
[11] A composition containing an auxin-degron system for controlling degradation of a protein of interest in a non-plant-derived eukaryotic cell, the composition including: a first nucleic acid encoding a TIR1 family protein; an auxin or an auxin analog having an affinity for the TIR1 family protein; a second nucleic acid encoding a fusion protein containing a degradation tag that contains at least a part of an Aux/IAA family protein and has an affinity for a complex of a TIR1 family protein with an auxin or an auxin analog, and a protein having an affinity for the target protein.
[12] The composition according to [11], in which the protein having the affinity for the target protein is a protein domain that binds to the target protein.
[13] The composition according to [12], in which the protein domain that binds to the target protein is a single-chain antibody.
[14] The composition according to any one of [11] to [13], in which the fusion protein is ubiquitination-resistant.
[15] The composition according to any one of [11] to [14], in which the TIR1 family protein is a rice-derived protein.
[16] The composition according to any one of [11] to [15], in which the TIR1 family protein is a mutant TIR1 family protein having a mutation at an auxin binding site.
[17] The composition according to [16], in which the mutant TIR1 family protein is a protein in which F at a position 74 of OsTIR1 has been mutated into A, G, or S.
[18] The composition according to any one of [11] to [17], in which the auxin analog is a compound represented by General Formula (I) or an ester thereof. 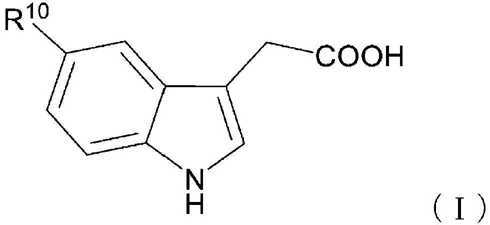 (In General Formula (I), R¹⁰ is a cyclic aliphatic hydrocarbon group which may have a substituent and in which a part of carbon atoms constituting a ring may be substituted with a heteroatom, or an aromatic hydrocarbon group which may have a substituent and in which a part of carbon atoms constituting a ring may be substituted with a heteroatom.)
[19] The composition according to any one of [11] to [18], further containing a third nucleic acid encoding a linker linked between the first nucleic acid and the second nucleic acid for controlling, by one promoter, a plurality of genes.
[20] The composition according to any one of [11] to [19], which is for pharmaceutical use.
[21] A fusion protein for use in an auxin-degron system for controlling degradation of a target protein in a non-plant-derived eukaryotic cell, the fusion protein containing a degradation tag that contains at least a part of an Aux/IAA family protein and has an affinity for a complex of a TIR1 family protein with an auxin or an auxin analog, and a protein having an affinity for the target protein.
[22] The fusion protein according to [21], in which the protein having the affinity for the target protein is a protein domain that binds to the target protein.
[23] The fusion protein according to [22], in which the protein domain that binds to the target protein is a single-chain antibody.
[24] The fusion protein according to any one of [21] to [23], in which the fusion protein is ubiquitination-resistant.
[25] The fusion protein according to [24], in which only the degradation tag is ubiquitination-resistant.
[26] A kit including an auxin-degron system for controlling degradation of a target protein in a non-plant-derived eukaryotic cell, the kit including a first nucleic acid encoding a TIR1 family protein, an auxin or an auxin analog having an affinity for the TIR1 family protein, and a second nucleic acid encoding the fusion protein according to any one of [21] to [25].
[27] The kit according to [26], in which the TIR1 family protein is a rice-derived protein.
[28] The kit according to [26] or [27], in which the TIR1 family protein is a mutant TIR1 family protein having a mutation at an auxin binding site.
[29] The kit according to [28], in which the mutant TIR1 family protein is a protein in which F at a position 74 of OsTIR1 has been mutated into A, G, or S.
[30] The kit according to any one of [26] to [29], in which the auxin analog is a compound represented by General Formula (I) or an ester thereof. 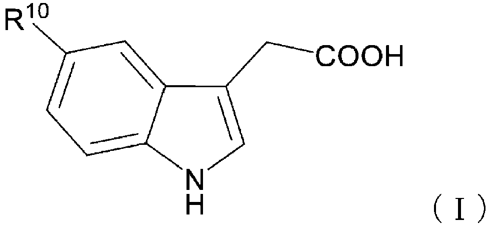 (In General Formula (I), R¹⁰ is a cyclic aliphatic hydrocarbon group which may have a substituent and in which a part of carbon atoms constituting a ring may be substituted with a heteroatom, or an aromatic hydrocarbon group which may have a substituent and in which a part of carbon atoms constituting a ring may be substituted with a heteroatom.)
[31] The kit according to any one of [26] to [30], further including a third nucleic acid encoding a linker linked between the first nucleic acid and the second nucleic acid for controlling, by one promoter, a plurality of genes.
[32] The kit according to any one of [26] to [31], in which the kit is for use in a treatment of a disease, and the kit is for simultaneous or sequential use in the treatment.
[33] A method for degrading a target protein using an auxin-degron system for controlling degradation of a target protein in a non-plant-derived eukaryotic cell, the method including expressing, in the eukaryotic cell, a first nucleic acid encoding a TIR1 family protein and a second nucleic acid encoding the fusion protein according to any one of [21] to [25]; and administering an auxin or an auxin analog having an affinity for the TIR1 family protein to the eukaryotic cell.
[34] The method for degrading a target protein according to [33], in which the auxin analog is a compound represented by General Formula (I) or an ester thereof. 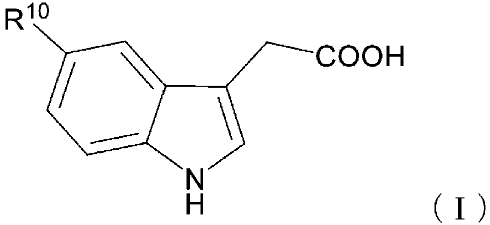 (In General Formula (I), R¹⁰ is a cyclic aliphatic hydrocarbon group which may have a substituent and in which a part of carbon atoms constituting a ring may be substituted with a heteroatom, or an aromatic hydrocarbon group which may have a substituent and in which a part of carbon atoms constituting a ring may be substituted with a heteroatom.)

### Advantageous Effects of Invention

According to the kit of the auxin-degron system of the present invention, the protein degradation can be controlled without adding a degradation tag to an endogenous target protein.

### BRIEF DESCRIPTION OF DRAWINGS

[FIG. 1] A schematic view illustrating a concept of the present invention.
[FIG. 2] A schematic view of a co-expression vector prepared in Example 1.
[FIG. 3] A schematic view illustrating the introduction of a co-expression vector containing an adapter (WT or KR adapter) that contains a gene encoding a GFP nanobody, together with an OsTIR1 (F74G) gene into cells expressing each of SMC6-GFP and DHC1-GFP in Example 1.
[FIG. 4] The result of the FACS analysis of the degradation of SMC6-GFP in Example 1.
[FIG. 5] The result of the FACS analysis of the degradation of DHC1-GFP in Example 1.
[FIG. 6] The result of Western blotting confirming the degradation of p53 in Example 2.
[FIG. 7] A schematic view of a co-expression vector prepared in Example 3.
[FIG. 8] A schematic view illustrating the introduction of a co-expression vector containing an adapter (WT, KR, or hybrid adapter) that contains a gene encoding a GFP nanobody, together with an OsTIR1 (F74G) gene into cells expressing each of SMC6-GFP, RAD21-GFP, and DHC1-GFP in Example 3.
[FIG. 9A] The result of the FACS analysis of the degradation of SMC6-GFP in Example 3. [FIG. 9B] The result of the FACS analysis of the degradation of RAD21-GFP in Example 3. [FIG. 9C] The result of the FACS analysis of the degradation of DHC1-GFP in Example 3.
[FIG. 10] The result of Western blotting confirming the degradation of p53 in Example 4.
[FIG. 11] The result of Western blotting confirming the degradation of RAS in Example 5.

### DESCRIPTION OF EMBODIMENTS

### <<Kit of auxin-degron system>>

A kit according to the present invention of an auxin-degron system for controlling degradation of a target protein in a non-plant-derived eukaryotic cell, the kit including: a first nucleic acid encoding a TIR1 family protein; an auxin or an auxin analog having an affinity for the TIR1 family protein; a second nucleic acid encoding a fusion protein containing a degradation tag that contains at least a part of an Aux/IAA family protein and has an affinity for a complex of a TIR1 family protein with an auxin or an auxin analog, and a protein having an affinity for the target protein.

An "auxin-degron system" is a protein degradation control technology developed by the present inventor, and is a system in which a plant-specific protein degradation system introduced by a plant hormone, an auxin, is applied to a non-plant-derived eukaryotic cell (for example, see Patent Documents 1 to 3).

Specifically, this system is a system in which a plant-derived TIR1 family protein as an F-box protein, which is a subunit of an E3 ubiquitin ligase complex (SCF complex), and a target protein labeled with a peptide consisting of a plant-derived Aux/IAA family protein or a partial sequence thereof are introduced into a non-plant-derived eukaryotic cell, whereby the TIR1 family protein, which is an auxin receptor, recognizes a peptide consisting of the Aux/IAA family protein or a partial sequence thereof and degrades the target protein using a ubiquitin/proteasome degradation system in the non-plant-derived eukaryotic cell in an auxin-dependent manner.

### <First nucleic acid>

In the present invention, the first nucleic acid encodes a TIR1 family protein.

The TIR1 family protein is an F-box protein which is one of subunits forming an E3 ubiquitin ligase complex (SCF complex) in protein degradation by an ubiquitin-proteasome system, and is a plant-specific protein. The TIR1 family protein serves as a receptor of an auxin which is a growth hormone, and is known to recognize an Aux/IAA family protein which is an inhibiting factor of an auxin communication system, by receiving an auxin, and thus to degrade a target protein.

The type of the gene encoding the TIR1 family protein is not limited as long as it is a gene encoding a plant-derived TIR1 family protein. In addition, the type of the plant from which the TIR1 family protein is derived is also not limited, and examples of the plant include Arabidopsis thaliana, rice, zinnia, pines, ferns, and Physcomitrella patens. Specific examples of the gene encoding the TIR1 family protein include a TIR1 gene, an AFB1 gene, an AFB2 gene, an AFB3 gene, an FBX14 gene, and an AFB5 gene.

Among these, an OsTIR1 gene, which is a TIR1 gene derived from rice, is preferable. Examples of such a gene include a gene having an accession number NM_001059194 (GeneID: 4335696) or an Os04g0395600 gene, or a gene having an accession number EAY93933 or OsI_15707, which is registered in NCBI, and more specific examples thereof include a gene consisting of a nucleotide sequence set forth in SEQ ID NO: 1. Furthermore, a gene consisting of a nucleotide sequence set forth in SEQ ID NO: 2, which is codon-optimized for human cells, is preferable.

In the present invention, from the viewpoint of preventing the target protein from being degraded in an auxin-independent manner, the TIR1 family protein is preferably a mutant TIR1 family protein having a mutation at an auxin binding site. Such a mutant protein is not particularly limited as long as it has an affinity for an auxin analog which will be described below, but is preferably a mutant protein in which the F at a position 74 of OsTIR1 has been mutated into A, G, or S, and more preferably a mutant protein in which the F at a position 74 has been mutated into G.

Specifically, the mutant TIR1 family protein is particularly preferably a protein that consists of a sequence including any one amino acid sequence of the following (a) to (c), and binds to the degradation tag through the complex with the auxin analog, leading to degradation of the target protein:
(a) an amino acid sequence in which an amino acid at a position 74 of an amino acid sequence set forth in SEQ ID NO: 3 is glycine,
(b) an amino acid sequence in which one to several amino acids are deleted, inserted, substituted, or added in a site other than the amino acid at a position 74 of (a), and
(c) an amino acid sequence having 80% or more identity in a site other than the amino acid at a position 74 of (a).

The number of amino acids deleted, inserted, substituted or added in (b) is preferably 1 to 120, more preferably 1 to 60, still more preferably 1 to 20, particularly preferably 1 to 10, and most preferably 1 to 5.

To be functionally identical to a protein consisting of sequences including the amino acid sequence of (a), the protein has 80% or more identity. With regard to such an identity, the identity is more preferably 85% or more, still more preferably 90% or more, particularly preferably 95% or more, and most preferably 99% or more.

Examples of the F74A protein of OsTIR1 include a protein consisting of the amino acid sequence set forth in SEQ ID NO: 4, and examples of a gene encoding the F74A protein of OsTIR1 include a gene consisting of the nucleotide sequence set forth in SEQ ID NO: 5. Examples of the F74G protein of OsTIR1 include a protein consisting of the amino acid sequence set forth in SEQ ID NO: 6, and examples of a gene encoding the F74G protein of OsTIR1 include a gene consisting of the nucleotide sequence set forth in SEQ ID NO: 7.

The first nucleic acid encoding the TIR1 family protein may be a DNA having an exon and an intron, or may be cDNA consisting of an exon. The first nucleic acid encoding the TIR1 family protein may be, for example, a full-length sequence in genomic DNA or a full-length sequence in a cDNA. In addition, the first nucleic acid encoding the TIR1 family protein may be a partial sequence in a genomic DNA or a partial sequence in a cDNA as long as an expressed protein functions as TIR1.

In the present specification, the phrase "functioning as the TIR1 family protein" means that, for example, the expressed protein recognizes the degradation tag (the full-length or partial protein of the Aux/IAA family protein) in the presence of the auxin analog. This is because the TIR1 family protein is capable of degrading a target protein labeled with the degradation tag as long as it is capable of recognizing the degradation tag.

### <Auxin analog>

In the present invention, the auxin analog is not particularly limited as long as it has an affinity for the mutant TIR1 family protein, and it is preferably a compound represented by General Formula (I) or an ester thereof.

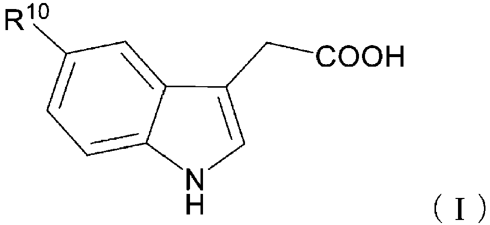

(In General Formula (I), R¹⁰ is a cyclic aliphatic hydrocarbon group which may have a substituent and in which a part of carbon atoms constituting a ring may be substituted with a heteroatom, or an aromatic hydrocarbon group which may have a substituent and in which a part of carbon atoms constituting a ring may be substituted with a heteroatom.)

### [Cyclic aliphatic Hydrocarbon group]

The cyclic aliphatic hydrocarbon group as R¹⁰ may be a monocyclic group or a polycyclic group.

Examples of the monocyclic aliphatic hydrocarbon include a cyclopropyl group, a cyclobutyl group, a cyclopentyl group, a cyclohexyl group, a methylcyclohexyl group, a dimethylcyclohexyl group, a cycloheptyl group, a cyclooctyl group, a cyclononyl group, and a cyclodecyl group.

Examples of the polycyclic alicyclic hydrocarbon group include a decahydronaphthyl group, an adamantyl group, a 2-alkyladamantan-2-yl group, a 1-(adamantan-1-yl)alkan-1-yl group, a norbornyl group, a methylnorbornyl group, and an isobornyl group.

In the cyclic aliphatic hydrocarbon group, some of carbon atoms constituting a ring may be substituted with heteroatoms. Examples of the heteroatom include an oxygen atom, a sulfur atom, and a nitrogen atom. Examples of such a heterocyclic ring include pyrrolidine, tetrahydrofuran, tetrahydrothiophene, piperidine, tetrahydropyran, tetrahydrothiopyran, dioxane, and dioxolane.

Examples of the substituent include an alkyl group having 1 to 6 carbon atoms, an alkoxy group having 1 to 6 carbon atoms, a halogen atom, and an aryl group having 6 to 30 carbon atoms.

Examples of the alkyl group include a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, an isopentyl group, a sec-pentyl group, a neopentyl group, a tert-pentyl group, a cyclopentyl group, a 2,3-dimethylpropyl group, a 1-ethylpropyl group, a 1-methylbutyl group, a 2-methylbutyl group, an n-hexyl group, an isohexyl group, a cyclohexyl group, a 2-methylpentyl group, a 3-methylpentyl group, a 1,1,2-trimethylpropyl group, and a 3,3-dimethylbutyl group.

Examples of the alkoxy group include an alkoxy group in which the R portion of -OR is the same as that of the above-described alkyl group having 1 to 6 carbon atoms. Among those, as the alkoxy group having carbon atoms, a methoxy group or an ethoxy group is preferable.

Examples of the halogen atom include a fluorine atom, a chlorine atom, a bromine atom, and an iodine atom. Among these, the fluorine atom or the chlorine atom is preferable as the halogen atom.

Examples of the aryl group include a phenyl group, a naphthyl group, a benzyl group, a phenethyl group, a biphenyl group, a pentalenyl group, an indenyl group, an anthranyl group, a tetracenyl group, a pentacenyl group, a pyrenyl group, a peryleneyl group, a fluorenyl group, and a phenanthryl group.

### [Aromatic hydrocarbon group]

Examples of the aromatic hydrocarbon group as R¹⁰ include the above-described aryl group having 6 to 30 carbon atoms.

In the aromatic hydrocarbon group, some of carbon atoms constituting a ring may be substituted with heteroatoms. Examples of the heteroatom include an oxygen atom, a sulfur atom, and a nitrogen atom. Examples of such a heterocyclic ring include pyrrole, furan, thiophene, pyridine, imidazole, pyrazole, oxazole, thiazole, pyridazine, pyrimidine, indole, benzimidazole, quinoline, isoquinoline, chromene, and isochromene.

Examples of the substituent include the same substituents as those mentioned in the section of [Cyclic aliphatic Hydrocarbon group].

The ester of the compound represented by General Formula (1) is a compound in which the hydrogen atom in -COOH of General Formula (I) is substituted with a hydrocarbon group or a -(CH₂)ₙ₁-O-C(=O)R¹² group (in the formula, n1 is an integer of 1 to 6, and R¹² is an alkyl group having 1 to 6 carbon atoms).

An ester substituted with an alkyl group as the hydrocarbon group is preferable.

Such a alkyl group is preferably an alkyl group having 1 to 6 carbon atoms and examples thereof include a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, an isopentyl group, a sec-pentyl group, a neopentyl group, a tert-pentyl group, a cyclopentyl group, a 2,3-dimethylpropyl group, a 1-ethylpropyl group, a 1-methylbutyl group, a 2-methylbutyl group, an n-hexyl group, an isohexyl group, a cyclohexyl group, a 2-methylpentyl group, a 3-methylpentyl group, a 1,1,2-trimethylpropyl group, and a 3,3-dimethylbutyl group.

An ester in which the hydrogen atom in -COOH of General Formula (I) is substituted with an acetoxy group as the -(CH₂)ₙ₁-O-C(=O)R¹² group, is preferable.

Among the compounds represented by General Formula (I), in which R¹⁰ is an aromatic hydrocarbon group, the compound represented by General Formula (I-1) or an ester body thereof is preferable.

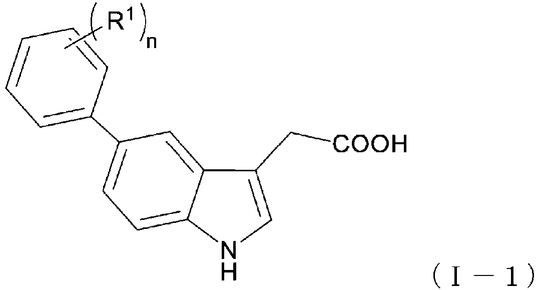

(In General Formula (1-1), R¹ is an alkyl group having 1 to 6 carbon atoms, an alkoxy group having 1 to 6 carbon atoms, a halogen atom, or an aryl group having 6 to 30 carbon atoms. n is an integer of 0 to 5, and in a case where n is an integer of 2 to 5, n pieces of R¹'s may be the same as or different from each other.)

Examples of the halogen atom of R¹ include a fluorine atom, a chlorine atom, a bromine atom, and an iodine atom. Among these, the fluorine atom or the chlorine atom is preferable as the halogen atom.

Examples of the alkyl group of R¹ include a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, an isopentyl group, a sec-pentyl group, a neopentyl group, a tert-pentyl group, a cyclopentyl group, a 2,3-dimethylpropyl group, a 1-ethylpropyl group, a 1-methylbutyl group, a 2-methylbutyl group, an n-hexyl group, an isohexyl group, a cyclohexyl group, a 2-methylpentyl group, a 3-methylpentyl group, a 1,1,2-trimethylpropyl group, and a 3,3-dimethylbutyl group. Among these, the methyl group or the ethyl group is preferable as the alkyl group having 1 to 6 carbon atoms.

Examples of the alkoxy group of R¹ include those of the alkoxy groups in which the R portion of -OR is the same as that of the above-described alkyl group having 1 to 6 carbon atoms. Among those, as the alkoxy group having carbon atoms, a methoxy group or an ethoxy group is preferable.

Examples of the aryl group of R¹ include a phenyl group, a naphthyl group, a benzyl group, a phenethyl group, a biphenyl group, a pentalenyl group, an indenyl group, an anthranyl group, a tetracenyl group, a pentacenyl group, a pyrenyl group, a peryleneyl group, a fluorenyl group, and a phenanthryl group.

n of R¹ is an integer of 0 to 5, and preferably 0 to 3. In a case where the compound represented by General Formula (I-1) has a plurality of R¹'s, examples thereof include the following compounds or esters thereof.

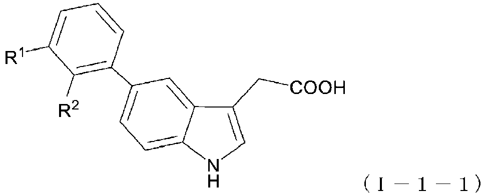

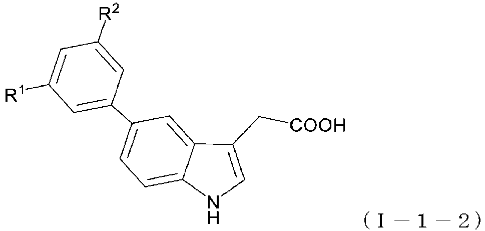

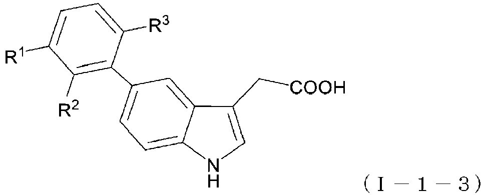

(In General Formulae (I-1-1) to (I-1-3), R¹ to R³ are each independently an alkyl group having 1 to 6 carbon atoms, an alkoxy group having 1 to 6 carbon atoms, a halogen atom, or an aryl group having 6 to 30 carbon atoms.)

In General Formulae (I-1-1) to (I-1-3), R¹ to R³ are the same as R¹ in General Formula (I-1).

In addition, among the compounds represented by General Formula (I), the following compounds or esters thereof are preferable.

A compound represented by Formula (I-1-4) (also referred to as 5-(3-MeOPh)-IAA).

A compound represented by Formula (I-1-5) (also referred to as 5-Ph-IAA).

A compound represented by Formula (I-1-6) (also referred to as 5-(3,4-diMePh)-IAA).

A compound represented by Formula (I-1-7) (also referred to as 5-(3-MePh)-IAA).

A compound represented by Formula (I-1-8) (also referred to as 5-(3-ClPh)-IAA).

A compound represented by Formula (I-1-10) (also referred to as 5-Ph-IAA-AM).

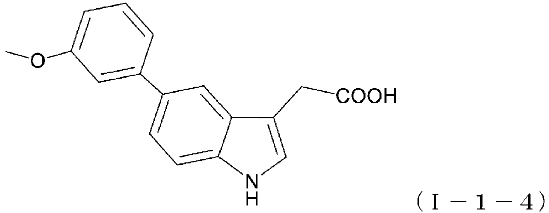

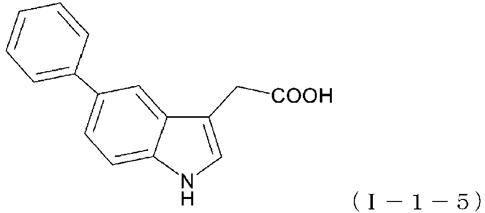

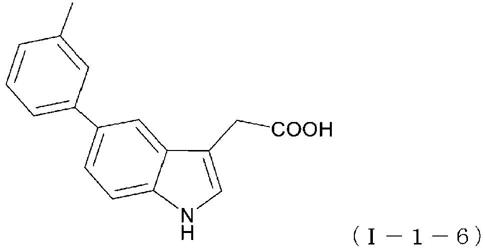

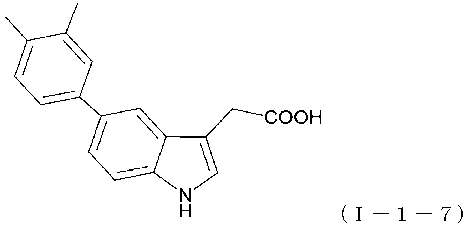

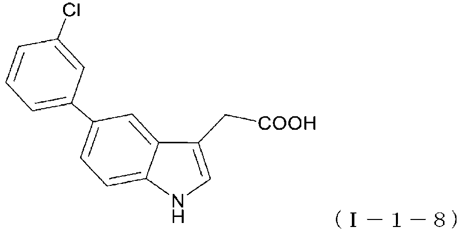

In addition, a compound represented by Formula (I-1-9) or an ester thereof is also preferable.

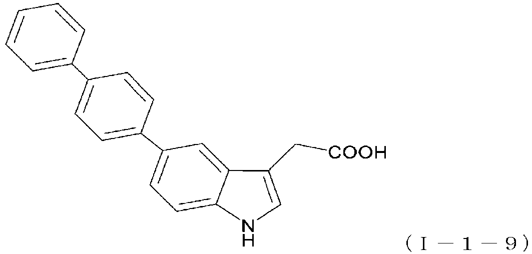

Furthermore, the compound represented by Formula (1-1-10) is also preferable.

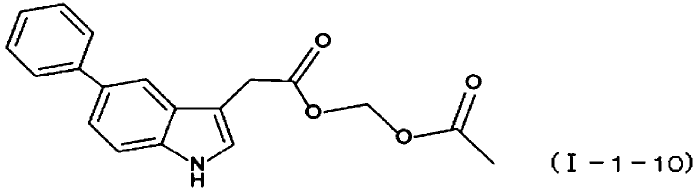

In addition, as the compound in which R¹⁰ is an aromatic hydrocarbon group, a compound represented by Formulae (I-2) to (I-5) or an ester thereof is also preferable.

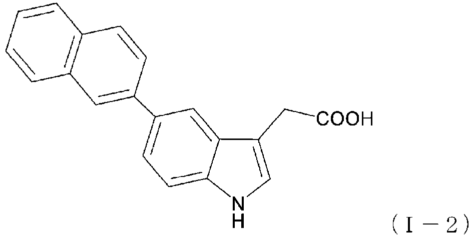

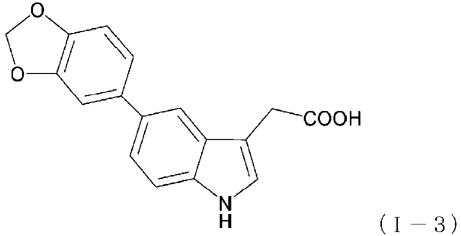

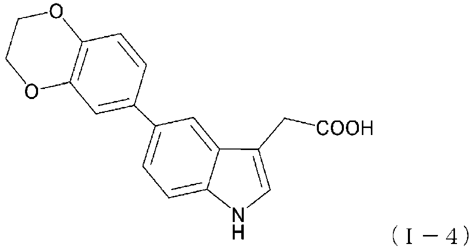

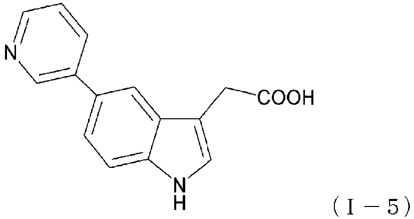

In addition, as the compound in which R¹⁰ is a cyclic aliphatic hydrocarbon group, a compound represented by General Formula (I-6) or an ester thereof is also preferable.

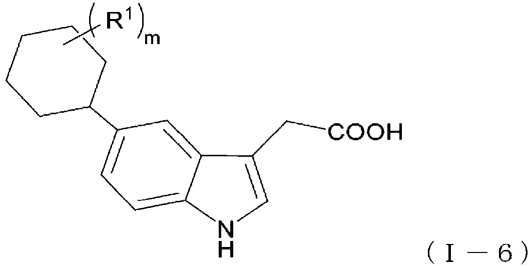

(In General Formula (I-6), R¹ is an alkyl group having 1 to 6 carbon atoms, an alkoxy group having 1 to 6 carbon atoms, a halogen atom, or an aryl group having 6 to 30 carbon atoms. m is an integer of 0 to 11, and in a case where m is an integer of 2 to 11, m pieces of R¹'s may be the same as or different from each other.)

R¹ is the same as that mentioned in General Formula (I-1). m is preferably 0 to 6, and more preferably 0 to 3.

In addition, as the compound in which R¹⁰ is a cyclic aliphatic hydrocarbon group, a compound represented by Formula (I-7) or an ester thereof is also preferable.

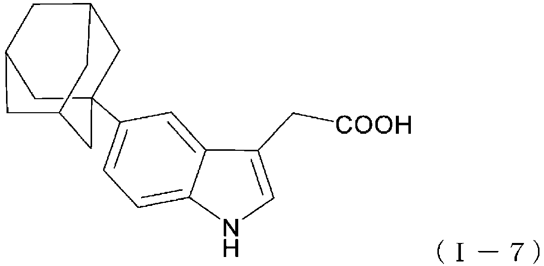

By using a combination of a mutant type TIR1 family protein and an auxin analog having high affinity for the mutant type TIR1 family protein, degradation of the target protein hardly occurs in the absence of auxin, and in the presence of auxin, an auxin-degron system can be provided in which the degradation efficiency of the target protein is very high even with a low concentration of the auxin analog.

### <Second nucleic acid>

In the present invention, the second nucleic acid codes for a fusion protein a degradation tag that contains at least a part of an Aux/IAA family protein and has an affinity for a complex of a TIR1 family protein with an auxin or an auxin analog, and a protein having an affinity for the target protein.

### [Degradation tag]

The type of a gene encoding the Aux/IAA family protein is not particularly limited as long as the gene is a plant-derived Aux/IAA family gene. Specific examples of the gene encoding the Aux/IAA family protein include an IAA1 gene, an IAA2 gene, an IAA3 gene, an IAA4 gene, an IAA5 gene, an IAA6 gene, an IAA7 gene, an IAA8 gene, an IAA9 gene, an IAA10 gene, an IAA11 gene, an IAA12 gene, an IAA13 gene, an IAA14 gene, an IAA15 gene, an IAA16 gene, an IAA17 gene, an IAA18 gene, an IAA19 gene, an IAA20 gene, an IAA26 gene, an IAA27 gene, an IAA28 gene, an IAA29 gene, an IAA30 gene, an IAA31 gene, an IAA32 gene, an IAA33 gene, and an IAA 34 gene.

The kit of the present invention may have any one type of full-length or partial sequence of the gene encoding the Aux/IAA family protein, or may have two or more types thereof. For example, the sequence of an Arabidopsis thaliana-derived Aux/IAA family gene is registered in the Arabidopsis Information Resource (TAIR), and the accession number of each gene is as follows.

The IAA1 gene (AT4G14560), the IAA2 gene (AT3G23030), the IAA3 gene (AT1G04240), the IAA4 gene (AT5G43700), the IAA5 gene (AT1G15580), the IAA6 gene (AT1G52830), the IAA7 gene (AT3G23050), the IAA8 gene (AT2G22670), the IAA9 gene (AT5G65670), the IAA10 gene (AT1G04100), the IAA11 gene (AT4G28640), the IAA12 gene (ATIG04550), the IAA13 gene (AT2G33310), the IAA14 gene (AT4G14550), the IAA15 gene (AT1G80390), the IAA16 gene (AT3G04730), the IAA17 gene (AT1G04250), the IAA18 gene (AT1G51950), the IAA19 gene (AT3G15540), the IAA20 gene (AT2G46990), the IAA26 gene (AT3G16500), the IAA27 gene (AT4G29080), the IAA28 gene (AT5G25890), the IAA29 gene (AT4G32280), the IAA30 gene (AT3G62100), the IAA31 gene (AT3G17600), the IAA32 gene (AT2G01200), the IAA33 gene (AT5G57420), and the IAA34 gene (AT1G15050).

Among these, the Arabidopsis thaliana IAA17 gene is preferable.

The degradation tag is not particularly limited as long as it is bound with the complex of the mutant TIR1 family protein and the auxin analog, leading to degradation of the target protein, and the degradation tag preferably includes a full-length or partial protein of the mAID among the Aux/IAA family proteins.

The "mAID" is the abbreviation of a "mini-auxin-inducible degron" and is a protein consisting of a partial sequence of the Arabidopsis thaliana IAA17, which is one of the Aux/IAA family proteins. This partial sequence is a sequence consisting of a region including at least two Lys residues on each of the N-terminal side and the C-terminal side of a domain II region of the Aux/IAA family protein or a sequence formed by linking two or more sequences described above. For example, the amino acid sequence of the mAID is represented by SEQ ID NO: 8.

### [Protein having an affinity for target protein]

In the present embodiment, by using the protein having an affinity for the target protein, it is not necessary to introduce the gene encoding the degradation tag into the chromosome by using genome editing or the like to label the target protein. The protein having an affinity for the target protein is not particularly limited as long as it captures the target protein and leads the target protein to degradation by the degradation tag. From the viewpoint of specificity, the protein having an affinity for the target protein is preferably a protein domain that binds to the target protein. Examples of the protein domain that binds to the target protein include a Fab fragment, a single-chain variable fragment (scFv), a nanobody, a monobody, and the like, and since a single-chain antibody is preferable, a nanobody derived from a camelid animal or a monobody derived from human fibronectin is more preferable. A screening method using phage display has been established for the nanobody and the monobody, and a platform capable of producing a single-chain antibody against any protein has been developed. In the present invention, by replacing the nucleic acid encoding the single-chain antibody, various proteins in the cell can be degraded at any timing.

### [Fusion protein]

In the kit of the present invention, the "auxin-degron system" is a system that degrades the target protein by utilizing the ubiquitin/proteasome degradation system. Therefore, from the viewpoint of efficiently degrading the target protein without degradation of the fusion protein itself by the ubiquitin/proteasome degradation system, the fusion protein preferably has ubiquitination resistance. Examples of a method of imparting the ubiquitination resistance include substituting a lysine residue as a target of ubiquitination with an arginine residue in the amino acid sequence constituting the fusion protein. In the amino acid sequence constituting the fusion protein having ubiquitination resistance, at least a part of the lysine residues are substituted with arginine residues, and it is preferable that all the lysine residues are substituted with arginine residues.

In addition, in the fusion protein, in a case where the function of the protein having an affinity for the target protein is impaired by substituting the amino acid of the protein, it is preferable that only the degradation tag has ubiquitination resistance. For example, the amino acid sequence of mAID in which all the lysine residues are substituted with arginine residues is set forth in SEQ ID NO: 13.

### <Third nucleic acid>

In the kit of the present invention, each of the first nucleic acid and the second nucleic acid may be DNA or mRNA in which a promoter sequence is operably linked, or may be incorporated into an expression vector, but the kit of the present invention may include a third nucleic acid encoding a linker linked between the first nucleic acid and the second nucleic acid for controlling, by one promoter, a plurality of genes.

Examples of the third nucleic acid include a nucleic acid encoding a readthrough linker and a nucleic acid encoding a non-readthrough linker.

Examples of the nucleic acid encoding a readthrough linker include a nucleic acid encoding a cleavage sequence by an endogenous enzyme, and a nucleic acid encoding a T2A peptide, a nucleic acid encoding a P2A peptide, a nucleic acid encoding an F2A peptide, and a nucleic acid encoding an E2A peptide.

Examples of the nucleic acid encoding a non-readthrough linker include an IRES.

The promoter is not particularly limited and can be appropriately determined depending on, for example, the type of a cell, or the like. Specific examples of the promoter include a CMV promoter, an SV40 promoter, an EF1a promoter, and an RSV promoter.

The expression vector is not particularly limited, and a vector known in the related art, such as a plasmid vector or a virus vector, can be used.

Examples of viral vectors include retroviral vectors, adenoviral vectors, Adeno-associated virus (AAV) vectors, herpes virus vectors, Sindbis virus vectors, and the like.

In the kit according to the embodiment of the present invention, the first nucleic acid to the third nucleic acid and the auxin or the auxin analog may be present as separate compositions. In addition, the first nucleic acid to the third nucleic acid and the auxin or the auxin analog may be present as one composition.

Such a composition preferably includes a pharmaceutically acceptable carrier. These composition can be administered, for example, orally in a form of a tablet, a coated tablet, a pill, a powder, a granule, a capsule, a liquid, a suspension, or an emulsion, or parenterally in a form of an injection agent, a suppository, or a skin external agent.

As the pharmaceutically acceptable carrier, one that is conventionally used for the formulation of a pharmaceutical composition can be used without particular limitation. More specific examples thereof include binders such as gelatin, cornstarch, gum tragacanth, and gum arabic; excipients such as starch and crystalline cellulose; swelling agents such as alginic acid; solvents for an injectable preparation, such as water, ethanol, and glycerin; and tacky adhesives such as a rubber-based tacky adhesive and a silicone-based tacky adhesive. One type of pharmaceutically acceptable carrier can be used singly, or two or more types thereof can be mixedly used.

In the kit according to the embodiment of the present invention, the composition of the present invention may further contain additives. Examples of the additives include lubricants such as calcium stearate and magnesium stearate; sweeteners such as sucrose, lactose, saccharin, and maltitol; flavorants such as a peppermint and a red oil; stabilizers such as benzyl alcohol and phenol; buffers such as phosphate and sodium acetate; dissolution aids such as benzyl benzoate and benzyl alcohol; antioxidants; and preservatives.

The additive can be used alone or in the form of a mixture of two or more types thereof.

The target for using the kit according to the embodiment of the present invention is not particularly limited as long as it is not a plant, and examples thereof include animals, fungi, protozoa, and the like. Examples of the animals include mammals such as humans, mice, rats and rabbits, fish or amphibians such as zebrafish and Xenopus laevis, and invertebrates such as C. elegans and Drosophila. In addition, cells derived from these can be used, and examples thereof include an established eukaryotic animal-derived cell, an ES cell, and an iPS cell. Specific examples of the eukaryotic cells include established human-derived cells, established mouse-derived cells, established chicken-derived cells, human ES cells, mouse ES cells, human iPS cells, and mouse iPS cells. Specific examples thereof include human HCT116 cells, human HT1080 cells, human NALM6 cells, human ES cells, human iPS cells, mouse ES cells, mouse iPS cells, and chicken DT40 cells. In addition, examples of the fungi include Saccharomyces cerevisiae and a fission yeast.

The kit according to the embodiment of the present invention is preferably a kit for treating a disease. The disease is not particularly limited, and examples thereof include cancers such as colorectal cancer, stomach cancer, and lung cancer; neurodegenerative diseases such as Alzheimer's disease and Parkinson's disease; and lifestyle-related diseases such as diabetes and arteriosclerosis. In the kit according to the embodiment of the present invention, the constituent may be used simultaneously or sequentially in a patient. In a case of simultaneous use, the first nucleic acid to the third nucleic acid and the auxin or the auxin analog are administered at once. In a case of sequential use, for example, the first nucleic acid to the third nucleic acid are administered to express a predetermined protein in a living body, and then the auxin or the auxin analog is administered to lead to degradation of the target protein in the living body.

### <<Gene therapy method>>

The gene therapy method according to the embodiment of the present invention is a gene therapy method for administering the first nucleic acid and the second nucleic acid, or the first nucleic acid, the second nucleic acid, and the third nucleic acid, and the auxin or the auxin analog to a patient.

The administration method in the gene therapy method according to the embodiment of the present invention is not particularly limited and may be appropriately determined depending on the symptom, body weight, age, sex, and the like of a patient. For example, a tablet, a coated tablet, a pill, a powder, a granular agent, a capsule agent, a liquid agent, a suspension, or an emulsion is administered orally. In addition, the injection preparation is administered singly or as a mixture with a general replacement fluid such as glucose and amino acids, and as necessary, an intraarterial, intramuscular, intradermal, subcutaneous, or intraperitoneal injection is administered.

A dosage of the pharmaceutical composition containing a nucleic acid in the gene therapy method according to the embodiment of the present invention varies depending on symptoms, body weight, age, gender, and the like of a patient, and thus it cannot be comprehensively determined. However, in a case of oral administration, it is sufficient for an active ingredient to be administered by, for example, 1 µg to 10 g per day, or for example, 0.01 to 2,000 mg per day. In the case of injection, for example, a daily dose thereof may be 0.1 µg to 1 g. For instance, a daily dose of the active ingredient may be 0.001 to 200 mg.

The administration period is preferably 1 to 10 days, and more preferably 3 to 7 days.

A method for administrating the auxin or the auxin analog is not particularly limited, and examples thereof include intraperitoneal administration, intravenous administration, intraarterial administration, intramuscular administration, intradermal administration, subcutaneous administration, and oral administration. The administration amount is, for example, preferably 0.1 mg/kg to 100 mg/kg, more preferably 0.2 mg/kg to 50 mg/kg, and still more preferably 0.5 mg/kg to 20 mg/kg per day. The administration period is preferably 1 to 10 days, and more preferably 3 to 7 days.

### [Example]

Hereinafter, the present invention will be described with reference to Examples, but the present invention is not limited to the following Examples.

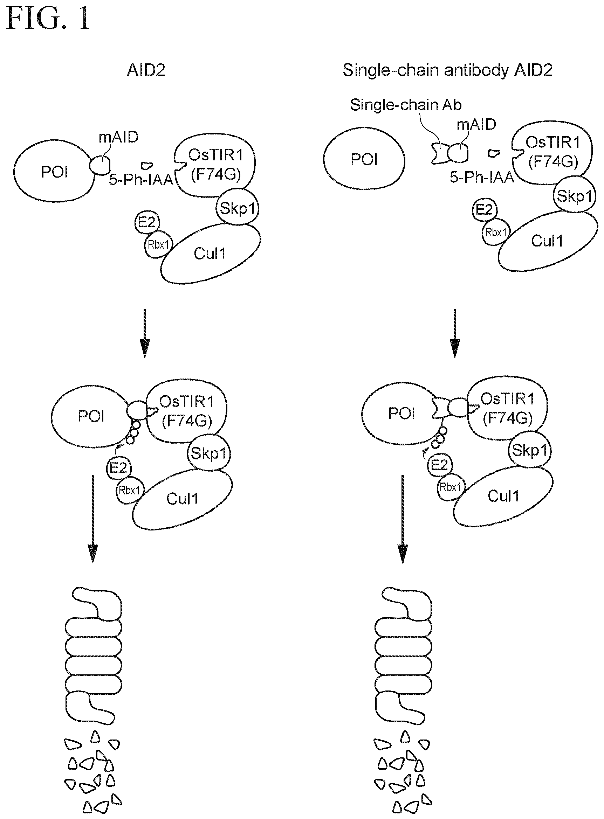

FIG. 1 is a schematic view illustrating a concept of the present invention. The left figure is a schematic view of a conventional auxin-degron system (AID2), and the right figure is a schematic view of an embodiment (single-chain antibody AID2) of the auxin-degron system according to the present invention. As described above, in the present invention, a fusion protein of a degradation tag and a protein (single-chain Ab in FIG. 1) having an affinity for a target protein is used, and the fusion protein introduced from the outside of the cell recognizes a POI (target protein) and leads to degradation.

### [Example 1]

### 1. Synthesis of auxin analog

A compound represented by Formula (I-1-5) (also referred to as 5-Ph-IAA) was synthesized.

### 2. Preparation of co-expression vector

As an mAID-single-chain antibody (nanobody) gene (also referred to as an adapter), a wild-type (WT adapter) and a ubiquitination-resistant mutant (KR adapter) in which all lysine residues (K) in the amino acid sequence encoded by the mAID-single-chain antibody (nanobody) gene were substituted with arginine residues (R) were prepared.

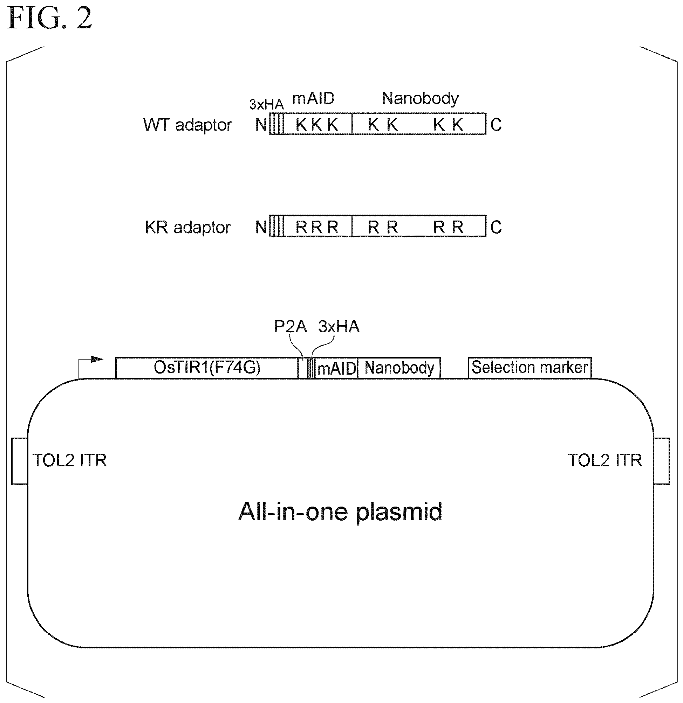

A co-expression vector in which the OsTIR1 (F74G) gene and the WT adapter or the KR adapter were connected by a self-cleaving sequence P2A gene was prepared (see FIG. 2).

### 3. Preparation of cells

In the chromosome of HCT116 cells, DNA encoding GFP was introduced into the downstream of DNA encoding each of the nuclear protein SMC6 and the cytoplasmic protein DHC1 by transposon, and cells expressing SMC6-GFP and cells expressing DHC1-GFP were prepared.

### 4. Introduction of adaptor and addition of auxin analog

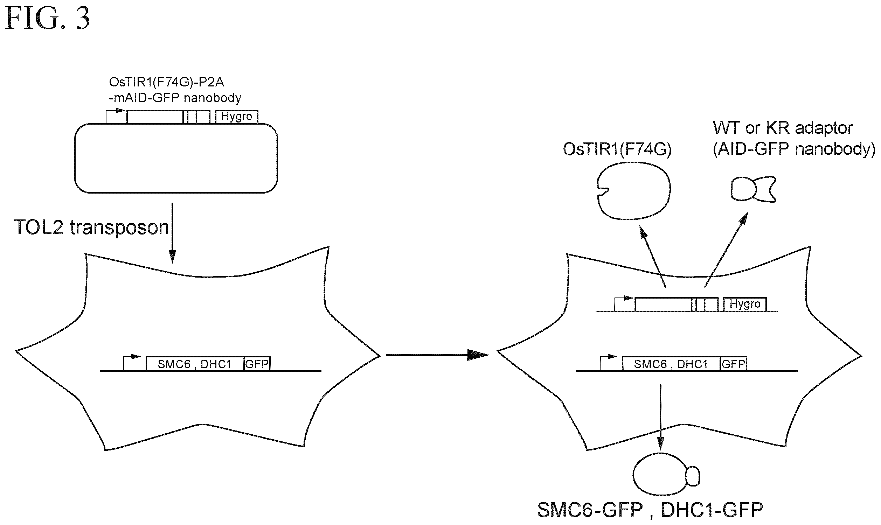

A co-expression vector containing an adapter (WT adapter; KR adapter) that contains a gene encoding a GFP nanobody (GFP nanobody WT gene sequence (SEQ ID NO: 9); GFP nanobody KR gene sequence (SEQ ID NO: 10)), together with the OsTIR1 (F74G) gene, was introduced into the cells expressing each SMC6-GFP and DHC1-GFP. As a control, a plasmid in which only OsTIR1 (F74G) and mAID were expressed was introduced (see FIG. 3).

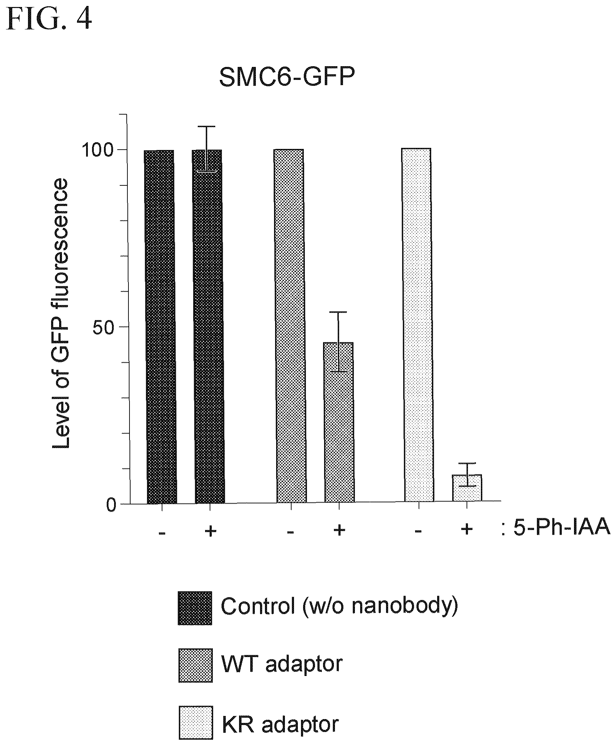

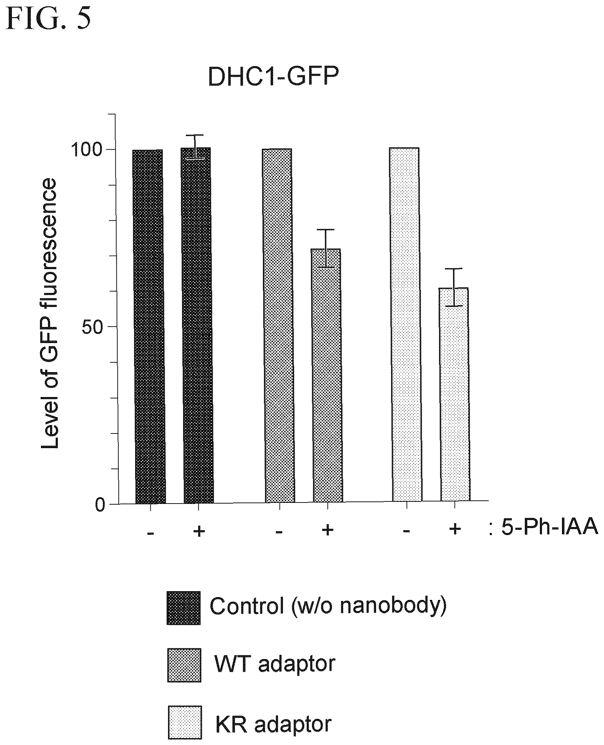

These cells were treated with 1 µM 5-Ph-IAA for 4 hours, and the GFP fluorescence in the cells was quantified using flow cytometry. The results are shown in FIGS. 4 and 5. It was confirmed that the target protein could be degraded and induced in a 5-Ph-IAA-dependent manner in the cells in which the adapter was expressed.

### [Example 2]

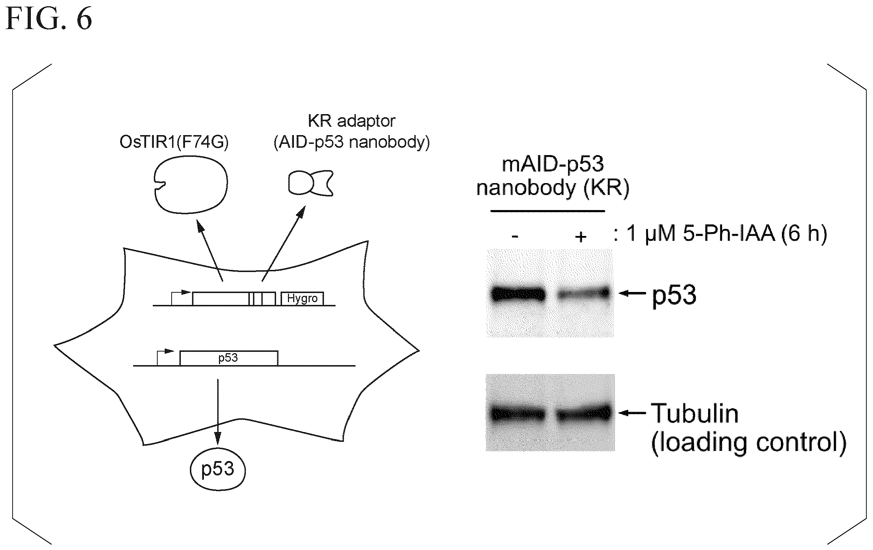

Furthermore, the same experiment was performed using an adapter (WT adapter; KR adapter) containing a gene encoding a p53 nanobody (p53 nanobody WT gene sequence (SEQ ID NO: 11); p53 nanobody KR gene sequence (SEQ ID NO: 12)) (see the left part of FIG. 6). The cells were treated with 1 µM 5-Ph-IAA for 6 hours, and the expression level of endogenous p53 was confirmed by Western blotting. The results are shown in the right part of FIG. 6. It was confirmed that the target protein could be degraded and induced in a 5-Ph-IAA-dependent manner in the cells in which the KR adapter was expressed.

These results confirmed that any target protein could be degraded and induced by using a single-chain antibody of the target protein.

### [Example 3]

### 1. Preparation of co-expression vector

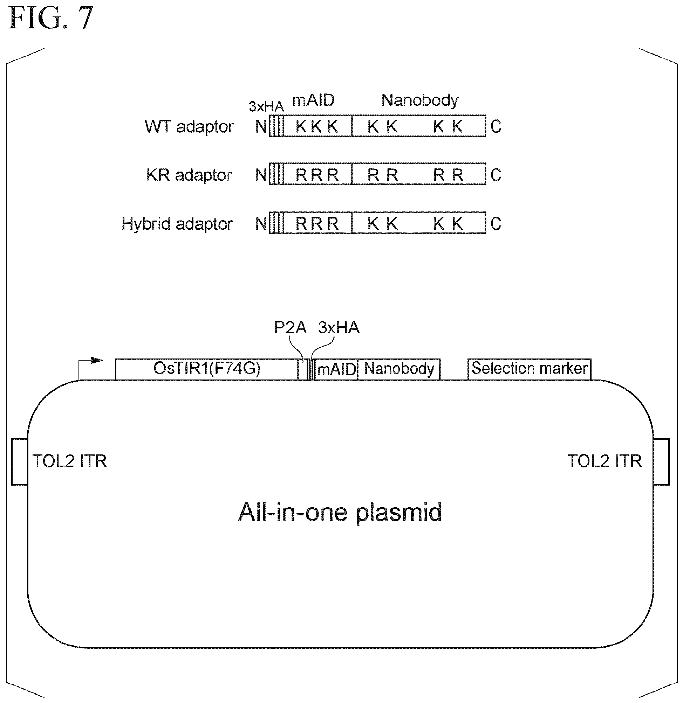

As the mAID-single-chain antibody (nanobody) gene, in addition to the WT adapter and the KR adapter, a hybrid adapter in which all lysine residues (K) in the amino acid sequence encoded by the mAID gene were substituted with arginine residues (R) was prepared. As in Example 1, a co-expression vector in which the OsTIR1 (F74G) gene and the WT adapter, the KR adapter, or the hybrid adapter were connected by a self-cleaving sequence P2A gene was prepared (see FIG. 7).

### 2. Preparation of cells

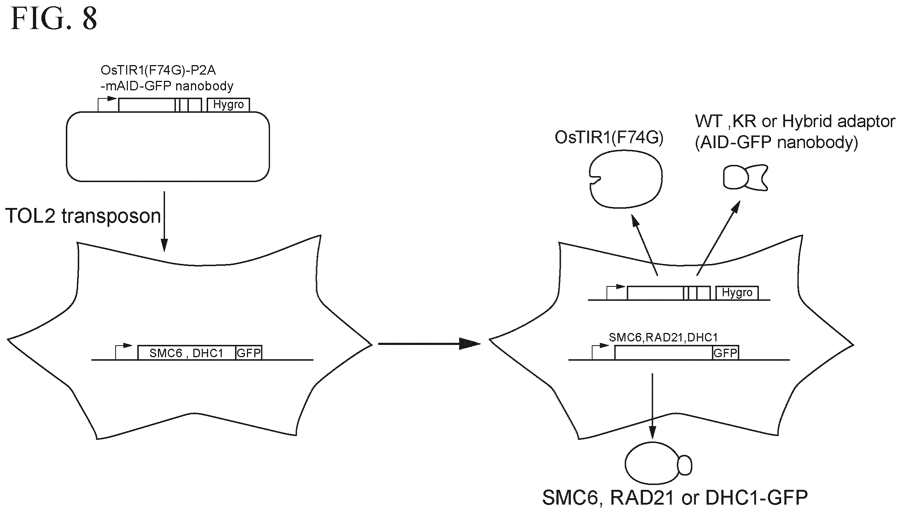

In the chromosome of HCT116 cells, DNA encoding GFP was added to the downstream of DNA encoding each of the nuclear protein SMC6, the nuclear protein RAD21, and the cytoplasmic protein DHC1 by genome editing, and cells expressing SMC6-GFP and cells expressing DHC1-GFP were prepared (see FIG. 8).

### 3. Introduction of adaptor and addition of auxin analog

A co-expression vector containing an adapter (WT adapter; KR adapter; hybrid adapter) that contains a gene encoding a GFP nanobody (GFP nanobody WT (SEQ ID NO: 9); GFP nanobody KR (SEQ ID NO: 10)), together with the OsTIR1 (F74G) gene, was introduced into the cells expressing each SMC6-GFP, RAD21-GFP, and DHC1-GFP by transposon. As a control, a plasmid in which only OsTIR1 (F74G) and mAID were expressed was introduced (see FIG. 8).

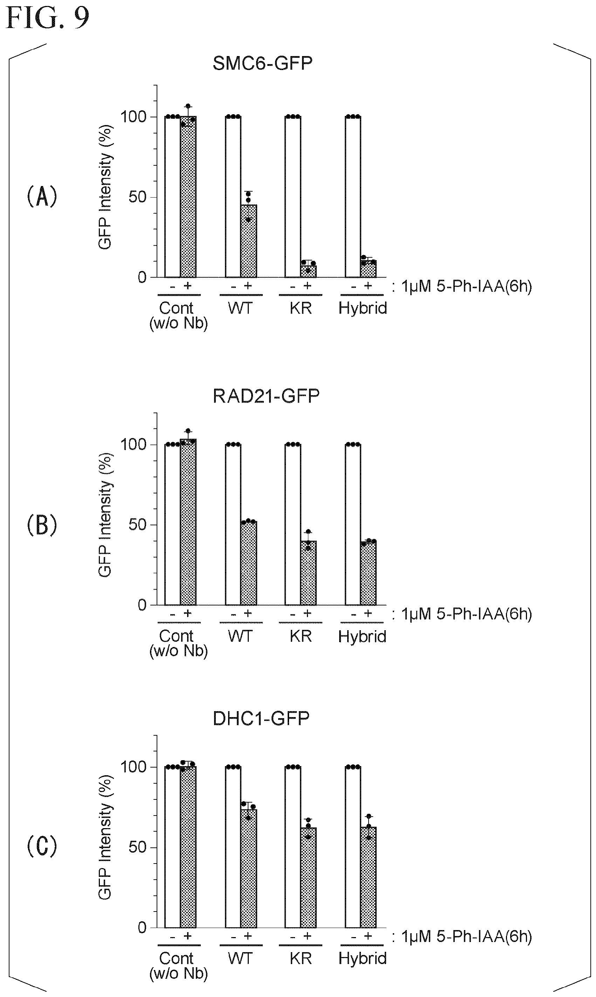

These cells were treated with 1 µM 5-Ph-IAA for 4 hours, and the GFP fluorescence in the cells was quantified using flow cytometry. The results are shown in FIG. 9. It was confirmed that the target protein could be degraded and induced in a 5-Ph-IAA-dependent manner in the cells in which the adapter was expressed. In particular, the degradation efficiency was excellent in the KR adapter and the hybrid adapter.

### [Example 4]

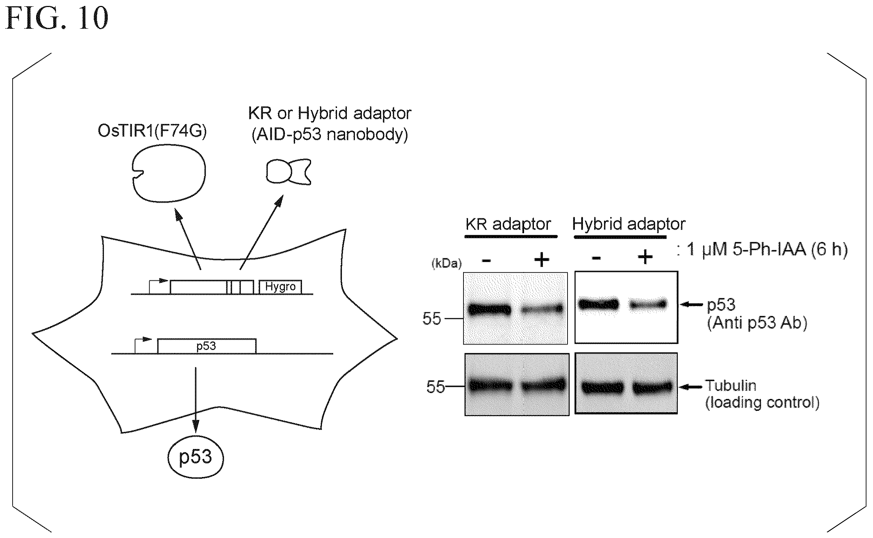

Furthermore, the same experiment was performed using an adapter (WT adapter; KR adapter; hybrid adapter) containing a gene encoding a p53 nanobody (p53 nanobody WT gene sequence (SEQ ID NO: 11); p53 nanobody KR gene sequence (SEQ ID NO: 12)) (see the left part of FIG. 10). The cells were treated with 1 µM 5-Ph-IAA for 6 hours, and the expression level of endogenous p53 was confirmed by Western blotting. The results are shown in the right part of FIG. 6. It was confirmed that the target protein could be degraded and induced in a 5-Ph-IAA-dependent manner in the cells in which the KR adapter was expressed.

### [Example 5]

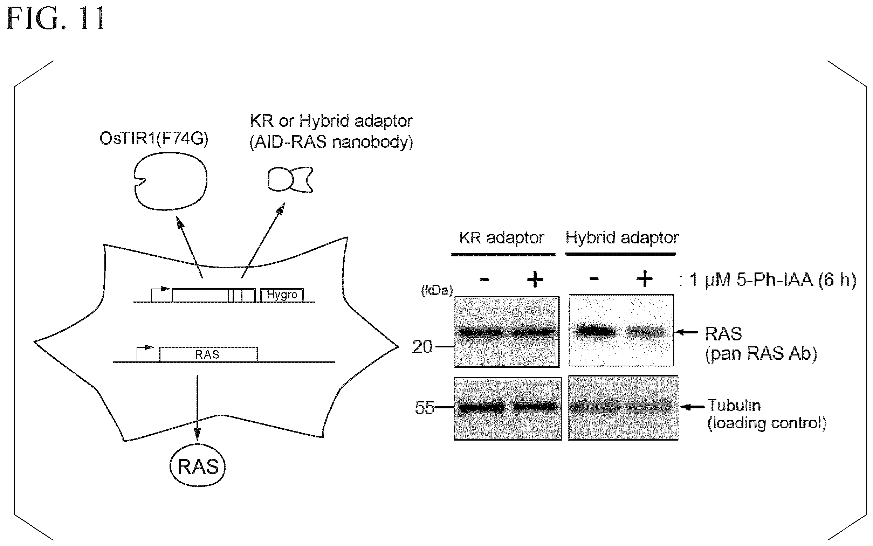

Furthermore, the same experiment was performed using an adapter (KR adapter; hybrid adapter) containing a gene encoding a RAS nanobody (gene sequence of RAS nanobody WT (SEQ ID NO: 14), amino acid sequence of RAS nanobody WT (SEQ ID NO: 15); gene sequence of RAS nanobody KR (SEQ ID NO: 16), amino acid sequence of RAS nanobody KR (SEQ ID NO: 17)) (see the left part of FIG. 11). The cells were treated with 1 µM 5-Ph-IAA for 6 hours, and the expression level of endogenous RAS was confirmed by Western blotting. The results are shown in the right part of FIG. 6. It was confirmed that the target protein could be efficiently degraded and induced in a 5-Ph-IAA-dependent manner in the cells in which the hybrid adapter was expressed.

### INDUSTRIAL APPLICABILITY

According to the auxin-degron system of the present invention, the protein degradation can be controlled without adding a degradation tag to an endogenous target protein.

## Claims

1. A fusion protein for use in an auxin-degron system for controlling degradation of a target protein in a non-plant-derived eukaryotic cell, the fusion protein comprising:
a degradation tag that contains at least a part of an Aux/IAA family protein and has an affinity for a complex of a TIR1 family protein with an auxin or an auxin analog; and
a protein having an affinity for the target protein.

2. The fusion protein according to Claim 1,
wherein the protein having the affinity for the target protein is a protein domain that binds to the target protein.

3. The fusion protein according to Claim 2,
wherein the protein domain that binds to the target protein is a single-chain antibody.

4. The fusion protein according to Claim 1,
wherein the fusion protein is ubiquitination-resistant.

5. The fusion protein according to Claim 1,
wherein only the degradation tag is ubiquitination-resistant.

6. A kit including an auxin-degron system for controlling degradation of a target protein in a non-plant-derived eukaryotic cell, the kit comprising:
a first nucleic acid encoding a TIR1 family protein;
an auxin or an auxin analog having an affinity for the TIR1 family protein; and
a second nucleic acid encoding the fusion protein according to any one of Claims 1 to 5.

7. The kit according to Claim 6,
wherein the TIR1 family protein is a rice-derived protein.

8. The kit according to Claim 6,
wherein the TIR1 family protein is a mutant TIR1 family protein having a mutation at an auxin binding site.

9. The kit according to Claim 8,
wherein the mutant TIR1 family protein is a protein in which F at a position 74 of OsTIR1 has been mutated into A, G, or S.

10. The kit according to Claim 6,
wherein the auxin analog is a compound represented by General Formula (I) or an ester thereof, 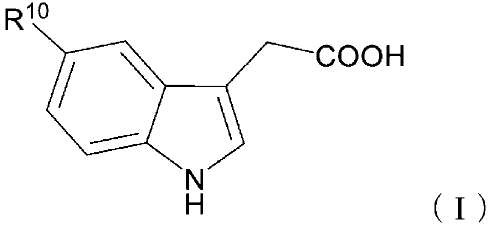
(in General Formula (I), R10 is a cyclic aliphatic hydrocarbon group which may have a substituent and in which a part of carbon atoms constituting a ring may be substituted with a heteroatom, or an aromatic hydrocarbon group which may have a substituent and in which a part of carbon atoms constituting a ring may be substituted with a heteroatom).

11. The kit according to Claim 6, further comprising:
a third nucleic acid encoding a linker linked between the first nucleic acid and the second nucleic acid for controlling, by one promoter, a plurality of genes.

12. The kit according to Claim 6,
wherein the kit is for use in a treatment of a disease, and
the kit is for simultaneous or sequential use in the treatment.

13. A method for degrading a target protein using an auxin-degron system for controlling degradation of a target protein in a non-plant-derived eukaryotic cell, the method comprising:
expressing, in the eukaryotic cell, a first nucleic acid encoding a TIR1 family protein and a second nucleic acid encoding the fusion protein according to any one of Claims 1 to 5; and
administering an auxin or an auxin analog having an affinity for the TIR1 family protein to the eukaryotic cell.

14. The method for degrading a target protein according to Claim 13,
wherein the auxin analog is a compound represented by General Formula (I) or an ester thereof, 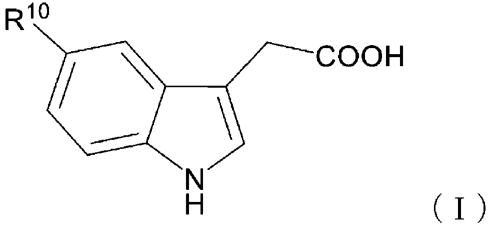
(in General Formula (1), R10 is a cyclic aliphatic hydrocarbon group which may have a substituent and in which a part of carbon atoms constituting a ring may be substituted with a heteroatom, or an aromatic hydrocarbon group which may have a substituent and in which a part of carbon atoms constituting a ring may be substituted with a heteroatom).
